# EUROPEAN PATENT SPECIFICATION

(11) **EP 1 235 523 B1**
(45) Date of publication and mention of the grant of the patent: **01.09.2004**
(21) Application number: 99974247.1
(22) Date of filing: 06.12.1999
(51) Int. Cl.: A61B 17/80

(54) **RESORBABLE BONE PLATE**
RESORBIERBARE KNOCHENPLATTE
PLAQUE VISSEE RESORBABLE

(43) Date of publication of application: 04.09.2002
(73) Proprietor: SYNTHES AG Chur, 7002 Chur (CH)
(72) Inventor: CURTIS, Raymond, CH-7260 Davos (CH); HEHLI, Markus, CH-7276 Davos (CH)
(74) Representative: Lusuardi, Werther Giovanni, Dr.
(86) International application number: PCT/CH1999/000582
(87) International publication number: WO 2001/041663

(56) References cited:
- EP-A- 0 530 585
- WO-A-97/09000
- WO-A-99/38448
- WO-A-99/44259
- US-A- 5 772 662
- DATABASE WPI Section PQ, Week 199307 Derwent Publications Ltd., London, GB; Class P31, AN 1993-054814 XP002144904 & JP 05 003883 A (TAKIRON), 14 January 1993 (1993-01-14)

## Description

This invention concerns a bone plate in accordance with the pre-characterising portion of Claim 1. Such a bone plate is disclosed in EP-A- 0530585, which defines the preamble of claim 1.

Bone plates made of titanium have shown that this material has such a bio-compatibility that soft-tissue is adhering thereto which can be in certain situations an undesirable property for bone plates. On the other hand bone plates made of bioresorbable materials usually have insufficient mechanical properties (e.g. stiffness).

The invention as claimed aims at overcoming the problems of adherence of soft-tissue and to optimize shape and function of an resorbable plate given the limitations of the properties of these materials.

The present invention provides a bone plate as defined in Claim 1.

Resorbable materials to be used for the device according to the invention can be resorbable polymers like highly purified polyhydroxyacids, polysaccharides, polyamines, polyaminoacids, polyorthoesters, polyanhydrides, polyamidoesters, polydioxanone, polyesteramides, copolyoxalates, polycarbonates or poly-(glutamic-co-leucine). Preferably polylactides are used or their combinations with polyhydroxybutyrates or polyhydroxyvalerates and/or resorbable glasses.
Other useful polyhydroxyacids comprise polycaprolactone, poly(L-lactide), poly(DL-lactide), polyglycolide, poly-(DL-lactide-co-glycolide), poly(DL-lactide-co-caprolactone).

Preferably the lower surface of the bone plate has a radius of curvature R which is smaller than 9 mm. The axis of the hollow cylinder corresponding to the curved plate is running parallel to the central longitudinal axis of the plate.

The ratio F/d of the total area F of the upper surface (including the surface area of the through holes) to the thickness d of the plate is in the range of 210 to 240 mm. Preferably the ratio F/f of the total area F of the upper surface (including the surface area of the through holes) to the true surface f of the upper surface (excluding the surface area of the through holes) is in the range of 1,10 - 1,25.

The width of the free ends of the longitudinal plate is narrowing to value b being preferably smaller than 0,15 B.

The upper surface of the bone plate has preferably a roughness of 0,5 µm to 5,0 µm, typically of 0,6 µm to 2,5 µm. in order to avoid the adherence of soft tissue after implantation. In any case it is preferred that the upper surface is smoother than the lower surface.

Preferably the bone plate is provided with a torsion around its central longitudinal axis which allows application to the required bone profile. The degree of torsion of the bone plate is preferably between ± 1° to ± 20° over the total length.

The length 1 of the central part of the longitudinal plate corresponds preferably to 73 - 82 % of the total length L of the plate.

In order to achieve better angular stability selected or all of the conical through holes may be provided with a thread. However, it is also possible to use through holes with a smooth inner surface.

The through holes have preferably a cone angle of 15° to 25°, typically of 18° to 22°.

The bone plate is provided preferably with n ≥ 4 holes (more preferably with n ≥ 6 holes), whereby the two most centrally located holes have preferably a distance which is at least 150 %, preferably 180 % of the distance x between the other through holes. When the central part of the plate is reinforced by omitting the most central hole (or holes) maximum rigidity of the cross section is obtained which is important for the bridging of bone defects.

The various features of novelty which characterize the invention are pointed out with particularity in the claims annexed to and forming part of this disclosure. For the better understanding of the invention, its operating advantages and specific objects attained by its use, reference should be had to the accompanying drawings, examples and descriptive matter in which are illustrated and described preferred embodiments of the invention.

In the drawings:
Fig. 1 is a view in elevation of the bone plate according to the invention; and
Fig. 2 is a section along line A-A of Fig. 1;

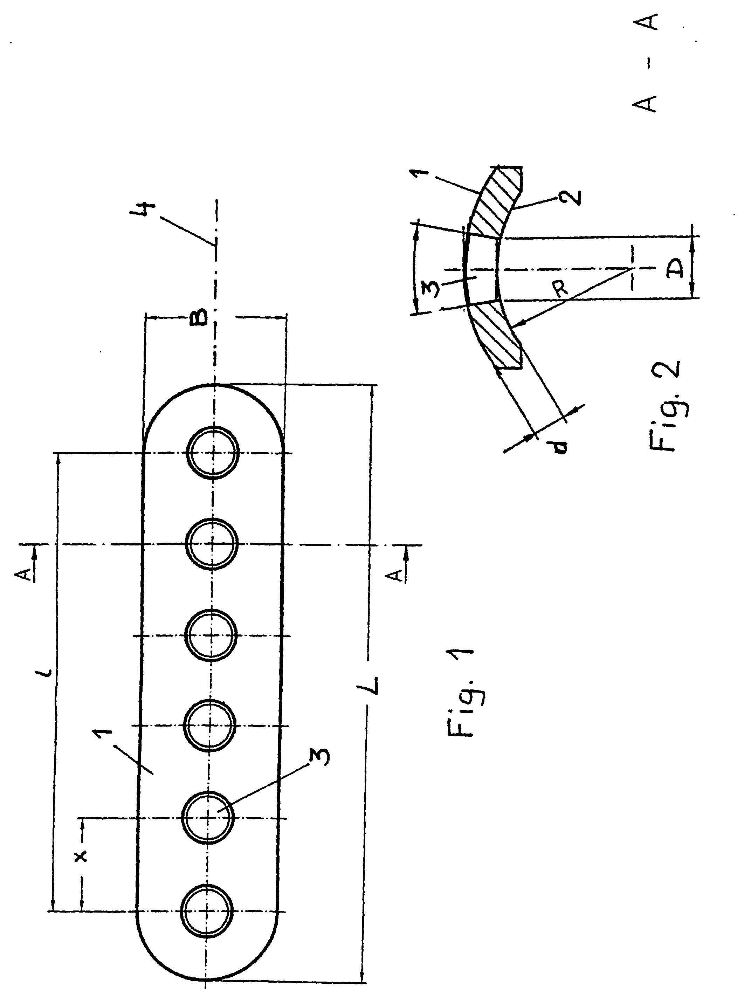

Figure 1 shows a bone plate of longitudinal shape having an upper curved surface 1, a curved lower surface 2 for contacting a long bone, e.g. a metacarpal bone, six conical through holes 3 connecting the upper surface 1 with the lower surface 2 for receiving bone screws and a central longitudinal axis 4.

The through holes 3 have a cone angle of 20° and a minimum diameter D of 2,8 mm located at the lower side 2.

The lower surface 2 has a radius of curvature R of 7 mm. The plate consists of polylactide copolymer with an enhanced degradation rate.
The ratio F/d of the total area F of the upper surface 1 (including the surface area of the through holes 3) to the thickness d of the plate has the value of 223 mm.
The ratio F/f of the total area F of the upper surface 1 including the surface area of the through holes 3 to the true surface f of the upper surface 1 excluding the surface area of the through holes 3 has the value of 1,17.

The central part of the longitudinal plate (over the length 1 in Fig. 1) has a constant width B whereas the width of the free ends of the longitudinal plate is narrowing gradually to value O in the form of a hemi-circle. The length 1 of the central part of the longitudinal plate corresponds to 77 % of the total length L of the plate.

## Claims

1. Bone plate of longitudinal shape having an upper surface (1), a curved lower surface (2) for contacting a bone, a plurality of conical through holes (3) connecting the upper surface (1) with the lower surface (2) for receiving bone screws, a central longitudinal axis (4) and consisting of a resorbable material,
**characterized in that**
A) the curved lower surface (2) has a radius of curvature R which is smaller than 10 mm;
B) the ratio F/d of the total area F of the upper surface (1) including the surface area of the through holes (3) to the thickness d of the plate is in the range of 190 to 270 mm; and
C) the central part of the longitudinal plate has a generally constant width B whereas the width of the free ends of the longitudinal plate is narrowing to value b < 0,3 B.

2. Bone plate according to claim 1, **characterized in that** the radius of curvature R is smaller than 9 mm.

3. Bone plate according to claim 1 or 2, **characterized in that** the ratio F/d is in the range of 210 to 240 mm.

4. Bone plate according to one of the claims 1 to 3, **characterized in that** the ratio F/f of the total area F of the upper surface (1) including the surface area of the through holes (3) to the true surface f of the upper surface (1) excluding the surface area of the through holes (3) is in the range of 1,10 to 1,25.

5. Bone plate according to one of the claims 1 to 4, **characterized in that** the width of the free ends of the longitudinal plate is narrowing to value b < 0,15 B.

6. Bone plate according to one of the claims 1 to 5, **characterized in that** the upper surface (1) has a roughness of 0,5 µm to 5,0, preferably of 0,6 µm to 2,5 µm.

7. Bone plate according to one of the claims 1 to 6, **characterized in that** the upper surface (1) is smoother than the lower surface (2).

8. Bone plate according to one of the claims 1 to 6, **characterized in that** plate is provided with a torsion around its central longitudinal axis (4).

9. Bone plate according to one of the claims 1 to 8, **characterized in that** the length 1 of the central part of the longitudinal plate corresponds to 73 to 82 % of the total length L of the plate.

10. Bone plate according to one of the claims 1 to 9, **characterized in that** conical through holes (3) are provided with a thread.

11. Bone plate according to one of the claims 1 to 9, **characterized in that** conical through holes (3) have a smooth inner surface.

12. Bone plate according to one of the claims 1 to 11, **characterized in that** it is provided with n ≥ 4 holes (3), preferably n ≥ 6 holes (3), and that the two most centrally located holes (3) have a distance which is at least 102 %, preferably of 150% to 180% of the distance x between the other holes (3).

13. Bone plate according to one of the claims 1 to 12, **characterized in that** the through holes (3) have a cone angle of 15° to 25°, preferably of 18° to 22°.

## Patentansprüche

1. Eine länglich geformte Knochenplatte aus resorbierbarem Material mit einer oberen Oberfläche (1), einer gekrümmten Unterfläche (2) für den Kontakt mit einem Knochen, mehreren konischen durchgehenden Löchern (3), die die obere Oberfläche (1) mit Unterfläche (2) verbinden und deren Funktion in der Aufnahme von Knochenschrauben besteht sowie einer zentralen Längsachse (4),
**dadurch gekennzeichnet dass**
a) die gekrümmte Unterfläche (2) einen Radius mit einer Krümmung R aufweist, der weniger als 10 mm beträgt;
b) Das Verhältnis F/d der Gesamtfläche F der oberen Oberfläche 1 (einschließlich der Fläche der durchgehenden Löcher(3) zur Höhe der Platte d hat einen Wert von 190-270 mm, und
c) der zentrale Teil der länglichen Platte hat im Normalfall eine konstante Breite B, wohingegen die freien Enden der Platte sich an einen Wert von b < 0,3 B annähern.

2. Eine Knochenplatte nach Anspruch 1, **dadurch gekennzeichnet, dass** der Radius der Krümmung R kleiner als 9 mm ist.

3. Eine Knochenplatte nach Anspruch 1 oder 2, **dadurch gekennzeichnet, dass** das Verhältnis F/d einen Wert von 210-240 mm hat.

4. Eine Knochenplatte nach einem der Ansprüche 1 bis 3, **dadurch gekennzeichnet, dass** das Verhältnis F/f der Gesamtfläche F der oberen Oberfläche (1) einschließlich der Oberfläche der durchgehenden Löcher (3) zur echten Oberfläche f der oberen Oberfläche (1) (ohne die Fläche der durchgehenden Löcher 3) einen Wert von 1,10 bis 1,25 hat.

5. Eine Knochenplatte nach einem der Ansprüche 1 bis 4, **dadurch gekennzeichnet, dass** die Breite der freien Enden der länglichen Platte sich dem Wert b < 0,15 B annähert.

6. Eine Knochenplatte nach einem der Ansprüche 1 bis 5, **dadurch gekennzeichnet, dass** die obere Oberfläche eine Rauheit von 0,5 - 5,0 µm, vorzugsweise von 0,6 - 2,5 µm aufweist.

7. Eine Knochenplatte nach einem der Ansprüche 1 bis 6, **dadurch gekennzeichnet, dass** die obere Oberfläche (1) glatter als die Unterfläche (2) ist.

8. Eine Knochenplatte nach einem der Ansprüche 1 bis, **dadurch gekennzeichnet, dass** die Platte um ihre zentrale Längsachse (4) gedreht ist.

9. Eine Knochenplatte nach einem der Ansprüche 1 bis 8, **dadurch gekennzeichnet, dass** die Länge des zentralen Teils der Längsplatte einen Anteil von 72 bis 82 % der Gesamtlänge L der Platte ausmacht.

10. Eine Knochenplatte nach einem der Ansprüche 1 bis 9, **dadurch gekennzeichnet, dass** einzelne durchgehenden Löcher (3) ein Gewinde haben.

11. Eine Knochenplatte nach einem der Ansprüche 1 bis 9, **dadurch gekennzeichnet, dass** einzelne durchgehenden Löcher (3) kein Gewinde haben.

12. Eine Knochenplatte nach einem der Ansprüche 1 bis 11, **dadurch gekennzeichnet, dass** sie n ≥ 4 Löcher (3), bevorzugt n ≥ 6 Löcher (3) hat, wobei die beiden Löcher (3), die dem Zentrum am nächsten sind, einen Abstand von 102 %, vorzugsweise von 150 - 180 % der Entfernung x zwischen den anderen durchgehenden Löchern (3) haben.

13. Eine Knochenplatte nach einem der Ansprüche 1 bis 12, **dadurch gekennzeichnet, dass** die durchgehenden Löcher (3) einen Konuswinkel von 15 ° - 25 °, vorzugsweise von 18 - 22 ° haben.

## Revendications

1. Plaquette pour les os de forme longitudinale comportant une face supérieure (1), une face inférieure courbée (2) destinée à entrer en contact avec l'os, une pluralité de perçages traversants coniques (3) reliant la face supérieure (1) à la face inférieure (2) pour recevoir des vis pour les os, un axe central longitudinal (4) et constitué d'un matériau résorbable,
**caractérisée en ce que**
A) la face inférieure courbée (2) a un rayon de courbure R inférieur à 10 mm,
B) le ratio F/d de la surface totale F de la face supérieure (1) comprenant la surfaces des perçages traversants (3) à l'épaisseur d de la plaquette est compris entre 190 et 270 mm, et
C) la partie centrale de la plaquette longitudinale a une largeur B généralement constante, la largeur des extrémités dégagées de cette plaquette longitudinale se rétrécissant pour atteindre une valeur b jusqu'à 0,3 B.

2. Plaquette pour les os selon la revendication 1, **caractérisée en ce que** le rayon de la courbure R est inférieur à 9 mm.

3. Plaquette pour les os selon la revendication 1 ou 2, **caractérisée en ce que** le ratio F/d est compris entre 210 et 240 mm.

4. Plaquette pour les os selon une des revendications 1 à 3, **caractérisée en ce que** le ratio F/f de la surface totale F de la face supérieure (1) comprenant la surface des perçages traversants (3) à la surface exacte f de la face supérieure (1), qui ne comprend pas la surface des trous traversants (3), est compris entre 1,10 à 1,25.

5. Plaquette pour les os selon une des revendications 1 à 4, **caractérisée en ce que** la largeur des extrémités dégagées de la plaquette longitudinale se rétrécit pour atteindre une valeur b jusqu'à 0,15 B.

6. Plaquette pour les os selon une des revendications 1 à 5, **caractérisée en ce que** la face supérieure (1) a une rugosité de 0,5 à 5,0 µm, de préférence de 0,5 à 2,5 µm.

7. Plaquette pour les os selon une des revendications 1 à 6, **caractérisée en ce que** la face supérieure (1) est plus lisse que la face inférieure (2).

8. Plaquette pour les os selon une des revendications 1 à 6, **caractérisée en ce que** la plaquette est pourvue d'une torsion autour de son axe central longitudinal (4).

9. Plaquette pour les os selon une des revendications 1 à 8, **caractérisée en ce que** la longueur I de la partie centrale de la plaquette longitudinale est égale à 73-82 % de la longueur totale L de la plaquette.

10. Plaquette pour les os selon une des revendications 1 à 9, **caractérisée en ce que** les perçages coniques traversants (3) sont pourvus d'un filetage.

11. Plaquette pour les os selon une des revendications 1 à 10, **caractérisée en ce que** les perçages coniques traversants (3) ont une surface intérieure lisse.

12. Plaquette pour les os selon une des revendications 1 à 11, **caractérisée en ce qu'**elle est pourvue d'un nombre n = 4 perçages (3), de préférence de n = 6 perçages (3) et que les deux perçages (3) situés le plus au centre ont une distance qui est au moins égale à 102 %, de préférence 150 à 180 % de la distance x entre les autres trous (3).

13. Plaquette pour les os selon une des revendications 1 à 12, **caractérisée en ce que** les trous traversants (3) ont un angle de cône de 15° à 25°, de préférence de 18° à 22°.
